# EUROPÄISCHE PATENTSCHRIFT

(11) **EP 2 238 934 B1**
(45) Veröffentlichungstag und Bekanntmachung des Hinweises auf die Patenterteilung: **21.12.2011**
(21) Anmeldenummer: 10162888.1
(22) Anmeldetag: 26.07.2002
(51) Int. Cl.: A61B 17/70

(54) **Knochenfixationseinrichtung und Schraube für eine solche**
Bone fixation device and screw for such
Dispositif de fixation d'os et vis destinée à celui-ci

(30) Priorität: 23.10.2001 EP 01125150
(43) Veröffentlichungstag der Anmeldung: 13.10.2010
(62) Teilanmeldung aus: 02754947.6
(73) Patentinhaber: Biedermann Motech GmbH, 78054 VS-Schwenningen (DE)
(72) Erfinder: Biedermann, Lutz, 78048, VS-Villingen (DE); Harms, Jürgen, 76227, Karlsruhe (DE); Ostermann, Peter, 46399, Bocholt (DE)
(74) Vertreter: Prüfer & Partner GbR European Patent Attorneys

(56) Entgegenhaltungen:
- FR-A- 2 702 361
- FR-A- 2 727 620
- US-A- 5 002 542
- US-A- 5 024 213
- US-A- 5 584 831
- US-B1- 6 179 838

## Beschreibung

Die Erfindung betrifft eine Einrichtung zur Fixation von Knochen, insbesondere am Becken oder von Röhrenknochen sowie Schrauben für solch eine Einrichtung.

Zur Fixation von Knochen, insbesondere am Becken oder von Röhrenknochen bei einer Fraktur werden in bekannter Weise Platten und Schrauben verwendet. Diese sind so ausgebildet, dass sie der Wölbung des zu fixierenden Knochens bzw. Beckenteiles einigermaßen anpassbar sind. Die Plattenform selbst und die Stellung der Schrauben hierzu sind nur in geringem Maß veränderbar.

Aus der US 5,002,542 ist eine Klemme für eine Knochenschraube bekannt, die zwei Abschnitte aufweist zur Aufnahme des Kopfes der Knochenschraube, einen Haken, der einen Stab aufnimmt und ein Klemmmittel, das die beiden Abschnitte zusammenhält, so dass sie den Kopf der Knochenschraube und den von dem Haken geführten Stab festklemmen. Durch die große Anzahl verschiedener Einzelteile ist diese Klemme jedoch sehr kompliziert zu handhaben und aufwändig in ihrer Herstellung.

Die US 5,584,831 zeigt eine Knochenfixationseinrichtung mit einer Knochenschraube und einer Klemme. Die Klemme besteht aus zwei Teilen, die auf der einen Seite kugelsegmentförmige Ausnehmungen zur Aufnahme des kugelförmigen Kopfs der Knochenschraube und auf der anderen Seite zylindersegmentförmige Ausnehmungen zur Aufnahme eines Stabes aufweisen. Die beiden Teile werden über eine Schraube so miteinander verbunden, dass sie einerseits den Kopf der Knochenschraube und andererseits den Stab festklemmen. Auch diese Fixationseinrichtung ist kompliziert zu handhaben und aufwändig in ihrer Herstellung.

Aufgabe der Erfindung ist es, eine Fixationseinrichtung und Schrauben für eine solche zu schaffen, mit denen eine Repositionierung und Fixation, z.B. eines Beckens oder von Röhrenknochen, wesentlich verbessert werden kann und insbesondere eine Fixationseinrichtung mit geringer Bauhöhe bereitzustellen. Weiterhin soll die Erfindung eine Fixationseinrichtung bereitstellen, die möglichst kostengünstig zu realisieren und möglichst einfach zu handhaben ist. Weiterhin soll die Erfindung eine Fixationseinrichtung bereitstellen, bei der die Fassung mit der Schraube gemeinsam eingesetzt werden kann. Weiterhin soll die Erfindung eine Fixationseinrichtung bereitstellen, die in der Lage ist, mehr als einen Stab zu fixieren.

Diese Aufgabe wird durch die in Patentanspruch 1 gekennzeichnete Fixationseinrichtung gelöst. Weiterbildungen der Erfindung sind in den Unteransprüchen gekennzeichnet.

Eine Fixationseinrichtung weist zumindest eine Schraube auf mit einem Schraubenelement und einer das Schraubenelement und einen Stab fixierenden Fassung, die ein Oberteil und ein Unterteil aufweist. Das Schraubenelement weist ein Gewindeschaftteil mit Gewinde und einen kugelförmigen Kopf auf. Oberteil und Unterteil der Fassung weisen auf der einen Seite kugelsegmentförmige Ausnehmungen zur Aufnahme des kugelförmigen Kopfs der Knochenschraube und auf der anderen Seite zylindersegmentförmige Ausnehmungen zur Aufnahme eines Stabes auf. Sie werden durch eine mit einem zwischen der kugelsegmentförmigen und der zylindersegmentförmigen Ausnehmung ausgebildeten Gewinde zusammenwirkenden Schraube miteinander verbunden, wodurch gleichzeitig der kugelförmige Kopf der Schraube und der Stab fixiert werden.

Vorzugsweise sind Oberteil und Unterteil als flache plattenförmige Teile mit identischem Aufbau ausgeführt und werden symmetrisch zu einer durch die Mittellinie des Stabs und den Mittelpunkt des kugelförmigen Kopfs der Schraube definierten Ebene angeordnet. Dadurch kann eine geringe Bauhöhe erzielt werden. Außerdem ist dadurch die Anzahl bereitzustellender Teile verringert, wodurch die Fixationseinrichtung kostengünstiger herzustellen und einfacher zu handhaben ist.

Bei einer identischen Ausführung von Oberteil und Unterteil entspricht der Ausnehmung zum Durchführen des Gewindeschaftteils im Unterteil eine Ausnehmung zum Durchführen eines Werkzeugs zum Drehen des Schraubelements im Oberteil. Dadurch kann die Schraube mit vormontierter Fassung in den Knochen eingeschraubt werden, was die Handhabung vereinfacht.

Vorzugsweise sind Oberteil und Unterteil der Fassung so ausgebildet, dass jeweils in der Mitte eine kugelsegmentförmige Ausnehmung zur Aufnahme des kugelförmigen Kopfs ausgebildet ist, zu beiden Seiten je ein Gewinde zum Verbinden von Oberteil und Unterteil ausgebildet ist und an den beiden Enden je eine zylindersegmentförmige Ausnehmung zur Aufnahme je eines Stabes ausgebildet ist. Auf diese Weise ist es möglich, mehr als einen Stab mit einer Schraube zu verbinden.

Weitere Merkmale und Zweckmäßigkeiten der Erfindung ergeben sich aus der Beschreibung von Ausführungsbeispielen anhand der Figuren. Von den Figuren zeigen:
- Fig. 1: eine seitliche Schnittdarstellung einer ersten Aus- führungsform;
- Fig. 2: eine perspektivische Darstellung einer zweiten Aus- führungsform;
- Fig. 3: eine seitliche Schnittdarstellung entlang Linie III- III in Fig. 2;
- Fig. 4: eine perspektivische Ansicht einer dritten Ausfüh- rungsform;
- Fig. 5: eine seitliche Schnittdarstellung entlang Linie III- III in Fig. 2 für eine vierte Ausführungsform;
- Fig. 6: eine perspektivische Ansicht einer fünften Ausfüh- rungsform.

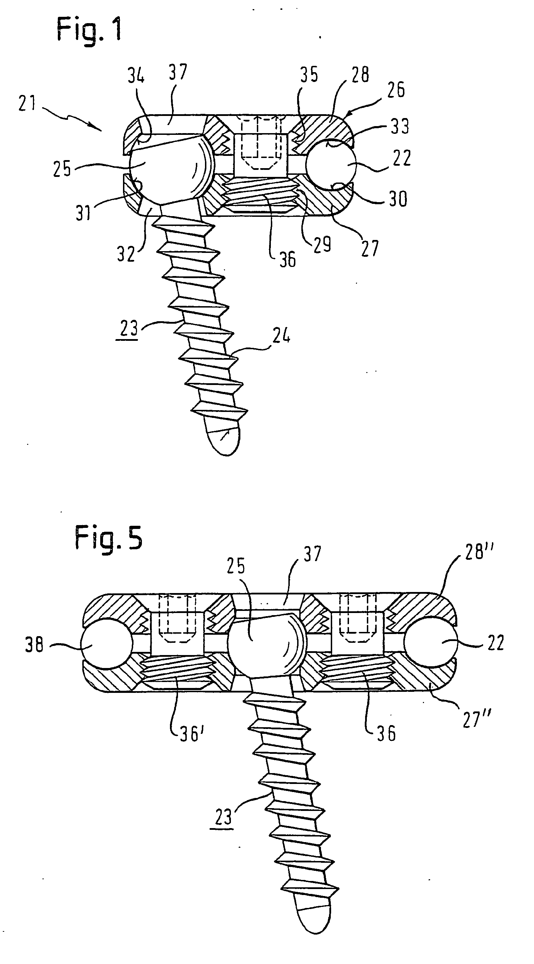

Zunächst wird mit Bezug auf Fig. 1 eine erste Ausführungsform der vorliegenden Erfindung beschrieben. Wie in Fig. 1 in eine seitlichen Schnittdarstellung gezeigt, weist eine Schraube 21 ein Schraubenelement 23 auf, welches ein Gewindeschaftteil 24 und einen sphärischen Kopf 25 aufweist, und eine Fassung 26, die ein Unterteil 27 und ein Oberteil 28 aufweist.

Unterteil 27 und Oberteil 28 sind identisch ausgebildet und werden symmetrisch zu einer durch die Mittellinie des Stabs und den Mittelpunkt des kugelförmigen Kopfs der Schraube definierten Ebene angeordnet.

Unterteil 27 und Oberteil 28 weisen je eine zentrale Bohrung auf, die mit einem Innengewinde 29, 35 versehen ist und auf der dem jeweils anderen Teil 27, 28 abgewandten Oberfläche eine Senkbohrung aufweist. Auf der einen Seite des zentralen Gewindes 29, 35 ist in einem Abstand von diesem eine zu dem jeweils anderen Teil 27, 28 hin zylindersegmentförmig ausgebildete Ausnehmung 30, 33 vorgesehen. Auf der dieser Ausnehmung gegenüberliegenden Seite des zentralen Gewindes 29, 35 weisen das unterteil 27 und das Oberteil 28 auf der dem jeweils anderen Teil 27, 28 zugewandten Seite je eine kugelsegmentförmige Ausnehmung 31, 34 auf. Auf der dem anderen Teil 27, 28 abgewandten Oberfläche schließt sich koaxial zu der Ausnehmung 31, 34 eine vorzugsweise nach außen hin zunehmende kegelförmig ausgebildete Ausnehmung 32, 37 an.

Ferner ist eine Schraube 36 vorgesehen, die durch das Innengewinde 35 des Oberteils einführbar und in das Innengewinde 29 des Unterteiles in der in Fig. 1 gezeigten Weise einschraubbar ist. Die Schraube hat in ihrem durch das Oberteil 28 geführten Teil einen Durchmesser, der kleiner ist als der Innendurchmesser des Innengewindes 35 des Oberteils und weist in ihrem durch das Unterteil 27 geführten Teil ein mit dem Innengewinde 29 des Unterteils zusammenwirkendes Außengewinde auf.

Wie am besten aus Fig. 1 ersichtlich ist, weisen die beiden Ausnehmungen 30 und 33 den gleichen Radius auf, der dem Radius eines aufzunehmenden Stabes 22 entspricht. Ferner weisen die beiden Ausnehmungen 31 und 34 den gleichen Radius auf, der dem Radius des Kopfes 25 entspricht. Die Ausnehmungen sind jeweils um den gemeinsamen Mittelpunkt angeordnet und so bestimmt, dass Oberteil 28 und Unterteil 27 in der in Fig. 1 gezeigten Position, in der Stab und Kopf aufgenommen sind, parallel zueinander sind und einen Abstand voneinander besitzen.

Im Betrieb sind Unterteil 27 und Oberteil 28 durch Losdrehen der Schraube 36 ausreichend weit entfernt und werden dann um 90° gegeneinander verdreht. Es kann dann zunächst das Schraubenelement 23 durch die vorzugsweise kegelförmig ausgebildete Ausnehmung 32 des Unterteils 27 eingeführt und mit Hilfe eines entsprechenden Werkzeuges in ein zu fixierendes Teil eingeschraubt werden. Der Kopf weist zu diesem Zweck eine entsprechende Ausnehmung zum Eingreifen mit einem Schraubendreher auf. Anschließend wird der Stab 22 aufgenommen. Dann wird das Oberteil 28 um 90° in die in Fig. 1 gezeigte Position zurückgedreht, und es erfolgt dann ein Verbinden durch Anziehen der Schraube 36 in der in Fig. 1 gezeigten fixierten Position, in der sowohl die Schraube als auch der Stab von dem Kopf fest gehalten und somit miteinander verbunden sind. Die vorzugsweise kegelförmig ausgebildete Ausführung der Ausnehmung 32 des Unterteils 27 macht es möglich, Schraubenelement 23 und Fassung 26 polyaxial ausrichtbar miteinander zu verbinden. Durch die Ausnehmung 37 des Oberteils kann ein Schraubendreher eingeführt werden, so dass das Schraubenelement 23 auch noch betätigt werden kann, wenn sie bereits mit der Fassung 26 verbunden ist.

Eine mit solchen Schrauben gebildete Fixationseinrichtung umfasst wenigsten zwei solcher Schrauben 21 und einen damit zu verbindenden Stab 22, so dass zwei miteinander zu verbindende oder zu fixierende Teile über die so eingeschraubten Schrauben und den Stab miteinander in einer vorgewünschten Position gehalten werden.

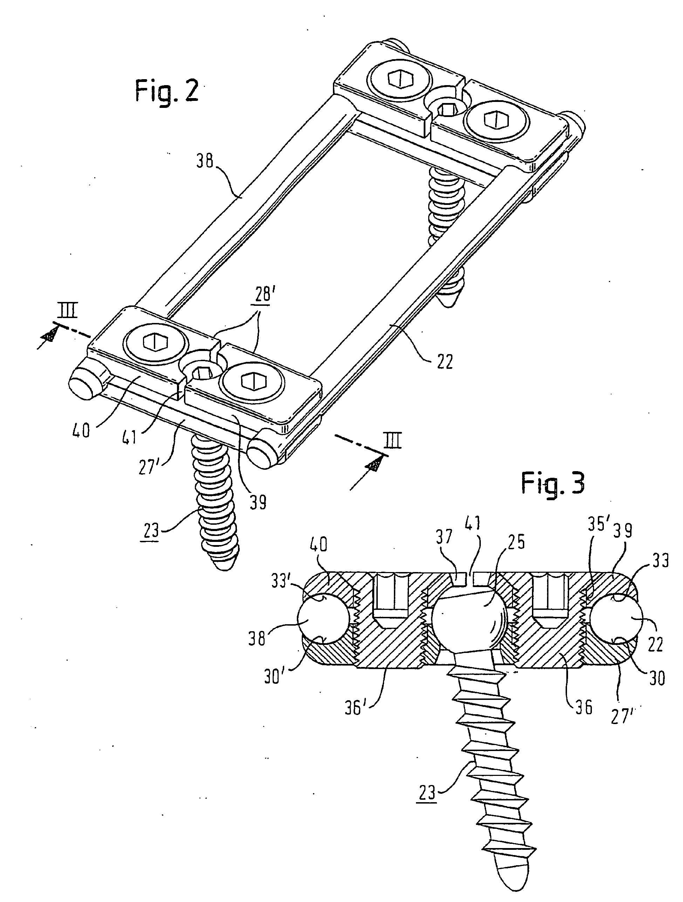

Zur Fixation mehrerer Knochenteile gegeneinander kann es zum Erhöhen der Stabilität erwünscht sein, die Knochenschraube mit mehr als einem Stab zu verbinden. In Fig. 2 und 3 ist eine zweite Ausführungsform der vorliegende Erfindung dargestellt, die in der Lage ist, zwei Stäbe mit dem Schraubenelement 23 zu verbinden.

Dazu ist die aus Unterteil 27' und Oberteil 28' bestehende Fassung auf eine dem ersten Stab 22 gegenüberliegende Seite verlängert zur Aufnahme eines zweiten Stabes 38. Diese Verlängerung kann so ausgeführt sein, dass Unterteil 27' und Oberteil 28' zu einer Ebene, die sich senkrecht zu der Ebene zwischen Oberteil und Unterteil und parallel zur Längsachse des Stabs 22 erstreckt und durch den Mittelpunkt der kugelsegmentförmigen Ausnehmungen (31, 34) verläuft, spiegelsymmetrisch ausgebildet sind, so dass auf der der Halterung für den Stab 22 gegenüberliegenden Seite eine gleichartige Halterung für einen zweiten Stab 38 vorgesehen ist. Zum Verklemmen ist wie die Schraube 36 zwischen dem Kopf 25 und dem Stab 22 eine entsprechende Schraube zwischen Kopf 25 und Stab 38 als Schraube 36' ausgebildet.

Unterteil 27' und Oberteil 28' sind in dieser Ausführungsform nicht identisch aufgebaut. Das Unterteil 27' weist beidseitig der kugelsegmentförmigen Aussparung 31 je eine Bohrung auf, die mit einem Innengewinde 29 versehen ist. Das Oberteil 28' weist koaxial zum Innengewinde 29 des Unterteils eine Bohrung 35' auf, die auf der dem Unterteil abgewandten Oberfläche eine Senkbohrung aufweist. Der Durchmesser der Bohrung 35' ist so gewählt, dass eine mit dem Innengewinde 29 zusammenwirkende Schraube durch die Bohrung hindurchführbar ist.

Wie aus den Fig. 2 und 3 ersichtlich ist, ist bei diesem Ausführungsbeispiel das Oberteil 28' so ausgebildet, dass die mit dem Kopf 25 und dem ersten Stab 22 zusammenwirkende erste Hälfte 39 und die mit dem Kopf 25 und dem zweiten Stab 38 zusammenwirkende zweite Hälfte 40 durch einen Schlitz 41 voneinander getrennt sind, der sich quer zu einer Linie erstreckt, die durch die Mittelpunkte der Schrauben 36, 36' bestimmt ist.

Die anderen Merkmale entsprechen denen der ersten Ausführungsform und werden an dieser Stelle nicht wiederholt.

Mit dieser Ausführungsform können gleichzeitig zwei Stäbe 22 und 38 erfasst werden, wodurch eine wesentlich größere Stabilisierung der Halterung für entsprechende Anwendungszwecke erreicht wird.

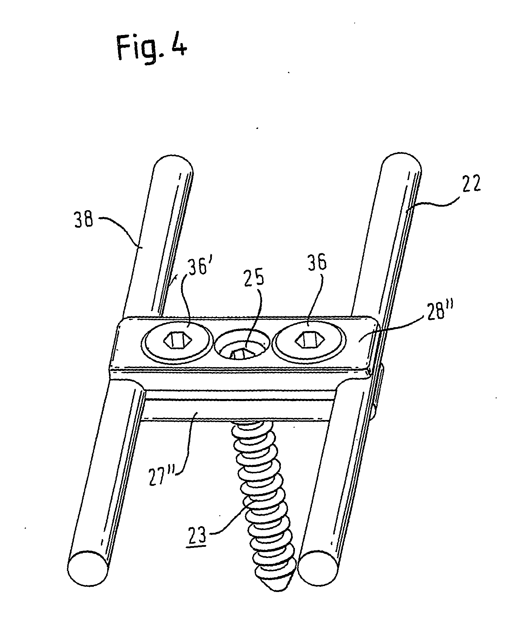

Eine in Fig. 4 dargestellte dritte Ausführungsform unterscheidet sich von der in Fig. 2 und 3 dargestellten zweiten Ausführungsform lediglich dadurch, dass das Oberteil 28" nicht geschlitzt ist.

Bei einer in Fig. 5 dargestellten vierten Ausführungsform sind auch bei der Ausführung zum Erfassen von zwei Stäben Oberteil und Unterteil identisch ausgebildet und werden symmetrisch zu einer durch die Mittellinie des Stabs 22 und den Mittelpunkt des kugelförmigen Kopfs 25 der Schraube definierten Ebene angeordnet.

Wie in der zweiten Ausführungsform sind Unterteil 27" und Oberteil 28" in sich symmetrisch ausgebildet mit der kugelförmigen Aussparung 31., 34 in der Mitte. Wie in der ersten Ausführungsform weisen sowohl das Unterteil 27" als auch das Oberteil 28" beidseitig der kugelförmigen Aussparung je eine Bohrung auf, die mit einem Innengewinde 29, 35 versehen ist und auf der dem jeweils anderen Teil 27", 28" abgewandten Oberfläche eine Senkbohrung aufweist. Die beiden Schrauben 36, 36' haben in ihrem durch das Oberteil 28" geführten Teil einen Durchmesser, der kleiner ist als der Innendurchmesser des Innengewindes 35 des Oberteils und weisen in ihrem durch das Unterteil 27 geführten Teil ein mit dem Innengewinde 29 des Unterteils zusammenwirkendes Außengewinde auf.

Die anderen Merkmale entsprechen denen der zweiten Ausführungsform und werden an dieser Stelle nicht wiederholt.

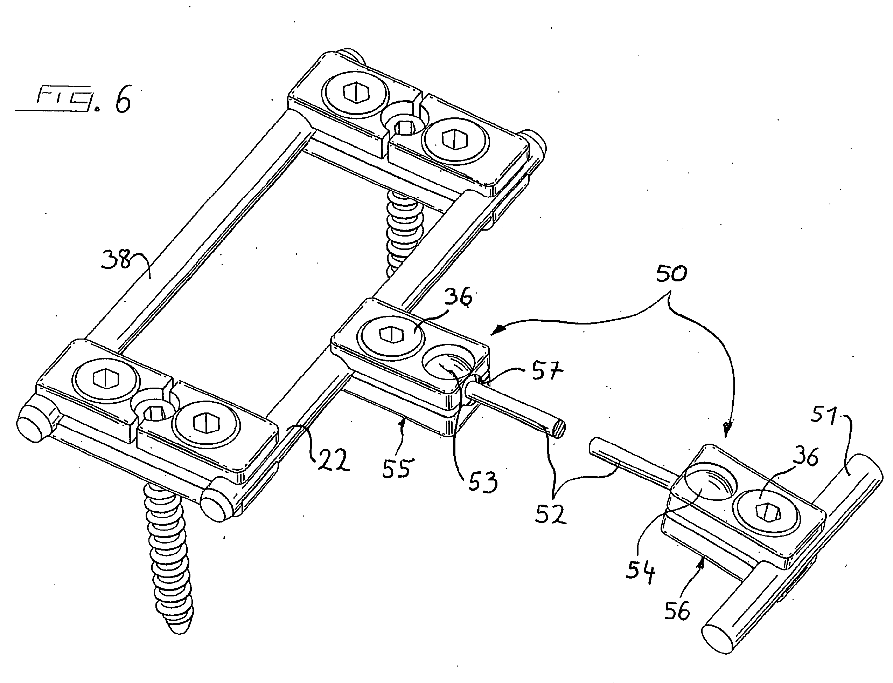

Eine in Fig. 6 dargestellte fünfte Ausführungsform stimmt bezüglich der zwei Stäbe 22, 38 und der diese verbindenden Fassung mit einer der in Fig. 2 bis 5 dargestellten Ausführungsformen vollständig überein. Fig. 6 zeigt zusätzlich eine Einrichtung 50 zum Verbinden eines weiteren Stabes 51 mit der Fixationseinrichtung. Die Einrichtung 50 weist einen als Schaft ausgebildeten Stab 52 auf, der gebrochen dargestellt ist und der an seinen beiden einander gegenüberliegenden Enden jeweils eine Kugel 53, 54 aufweist und an jedem seiner beiden Enden eine Fassung 55, 56, die in dem gezeigten Ausführungsbeispiel identisch aufgebaut sind. Diese entsprechen in ihrem Aufbau der in Fig. 1 dargestellten Fassung 26 mit der Ausnahme, dass zusätzlich zu den vorzugsweise kegelförmigen Ausnehmungen 32 und 37 eine ebenfalls vorzugsweise nach außen hin zunehmende kegelförmig ausgebildete Ausnehmung 57 vorgesehen ist, deren Mittelachse durch den Mittelpunkt des für den Kopf 53, 54 gebildeten Lagers geht, das dem den Kopf 25 aufnehmenden Lager in Fig. 1 entspricht, und in dem gezeigten Ausführungsbeispiel in der Ebene zwischen Oberteil und Unterteil liegt und nicht nur durch die Mitte der Kugel 53, 54, sondern auch durch die Mittelachse der Schraube 36 läuft.

Die Abmessungen zwischen Kugel 53 bzw. 54 und dem Lager ist so bemessen, dass bei leichter Lockerung der Schraube 36 einerseits ein Verschieben am Stab 22 bzw. 51 möglich ist, andererseits um die Längsachse der vorzugsweise kegelförmig ausgebildeten Öffnung 57 ein um einen Kegel schwenkbares Bewegen des Schaftes 52 möglich ist, so dass auch nicht parallele Stäbe 22, 51 dieser Vorrichtung verbindbar sind. Nach dem verbinden wird die Schraube 36 festgezogen, wodurch die Fassung sowohl die Stäbe 22 bzw. 51 als auch die Kugelköpfe 53 bzw. 54 fest umfasst.

In dem in Fig. 6 gezeigten Ausführungsbeispiel ist die sphärische Ausnehmung 34 (Fig. 1) zur Oberseite des Oberteils 28 hin offen. Es ist aber genauso gut möglich, Oberteil und Unterteil ohne die Ausnehmungen 32, 37 auszubilden, so dass die sphärischen Ausnehmungen 31, 34 zur Außenseite hin geschlossen ausgebildet sind.

In Fig. 6 ist die Verbindungseinrichtung 50 im Zusammenhang mit einer der in Fig. 2 bis 5 dargestellten Ausführungsformen mit zwei Stäben 22, 38 gezeigt. Die Verbindungseinrichtung 50 kann aber ebenso gut gemeinsam mit der in Fig. 1 mit einem einzelnen Stab 22 dargestellten Ausführungsform verwendet werden.

Die Vorrichtung weist gegenüber dem eingangs erwähnten Stand der Technik die Vorteile auf, dass sie weniger Bauteile benötigt, und daher kostengünstiger herzustellen und einfacher zu handhaben. Da der Stab und die Schraube gleichzeitig fixiert werden, ist eine einfachere Justierung und somit insgesamt eine einfachere Handhabung gegeben. Ferner besteht der Vorteil einer extrem niedrigen Bauhöhe. Die Schraube kann mit vormontierter Fassung in den Knochen eingeschraubt werden, was die Handhabung weiter vereinfacht. Weiterhin ist es möglich, mehr als einen Stab mit einer Schraube zu verbinden. Die Vorrichtung kann auch dazu verwendet werden, Stäbe untereinander zu verbinden.

## Patentansprüche

1. Einrichtung zur Fixation von Knochen und insbesondere eines Beckens, mit wenigstens einer Schraube (21) für eine Fixationseinrichtung mit:
einem Schraubenelement (23) mit einem Gewindeschaft (24) und einem kugelsegmentförmigen Kopf (25),
einer den Kopf (25) aufnehmenden Fassung (26) mit einem der Schaftseite zugewandten Unterteil (27') und einem der Schaftseite abgewandten Oberteil (28; 28'; 28"), die zusammen einen Stab (22; 38) umfassen können, und
einem Schraubenelement (36; 36') zum Verbinden der beiden Teile (27', 28') und gleichzeitigem Fixieren des Schraubenelements (23) mit kugelsegmentförmigem Kopf und des Stabes (22; 38); wobei
in dem Unterteil (27') ein Gewinde (29, 35) zum Zusammenwirken mit dem die beiden Teile verbindenden Schraubenelement (36; 36') vorgesehen ist, und in dem Oberteil (28') eine Bohrung (35; 35') koaxial zum Gewinde (29, 35) vorgesehen ist;
jeweils auf einer Seite des Gewindes (29, 35) und der Bohrung (35; 35') einander zugewandte zylindersegmentförmige Ausnehmungen (30'; 33') zum Aufnehmen des Stabes (22; 38) vorgesehen sind,
auf der jeweils gegenüberliegenden Seite des Gewindes (29, 35) und der Bohrung (35; 35') einander zugewandte kugelsegmentförmige Ausnehmungen (31, 34) zur Aufnahme des Kugelkopfes (25) vorgesehen sind,
**dadurch gekennzeichnet, dass**
zwischen den kugelsegmentförmigen Ausnehmungen (31, 34) und den einander abgewandten Deckflächen der beiden Teile (27', 28') Ausnehmungen (32, 37) vorgesehen sind, zum Durchführen des Gewindeschaftes (24) einerseits und eines Werkzeuges zum Drehen des Schraubenelements (23) mit kugelsegmentförmigem Kopf andererseits.

2. Fixationseinrichtung mit wenigstens zwei Schrauben (21) nach Anspruch 1 und einem von den Schrauben zu erfassenden Stab (22; 38).

3. Fixationseinrichtung nach Anspruch 1, wobei
die den Kopf (25) aufnehmende Fassung (26) mit dem der Schaftseite zugewandten Unterteil (27') und dem der Schaftseite abgewandten Oberteil (28'), einen ersten Stab (22) und einen zweiten Stab (38) umfassen kann, und wobei
zwei Schraubenelemente (36, 36') zum Verbinden der beiden Teile (27', 28') und gleichzeitigem Fixieren des Schraubenelements (23) mit kugelsegmentförmigem Kopf und der Stäbe (22, 38) vorgesehen sind.

4. Fixationseinrichtung nach Anspruch 3, **dadurch gekennzeichnet, dass** das Unterteil (27') und das Oberteil (28') in jeweils ihrer Mitte einander zugewandte kugelsegmentförmige Ausnehmungen (31, 34) zur Aufnahme des Kugelkopfes (25) aufweisen und um eine durch den Mittelpunkt der kugelsegmentförmigen Ausnehmungen (34, 31) sich erstreckenden Ebene, die sich senkrecht zu der Ebene zwischen Oberteil und Unterteil und parallel zur Längsachse des Stabs (22) erstreckt, in sich spiegelsymmetrisch ausgebildet sind.

5. Fixationseinrichtung nach Anspruch 3 oder 4, **dadurch gekennzeichnet, dass** das Unterteil (27') und das Oberteil (28') beidseitig der kugelsegmentförmigen Ausnehmungen (31, 34) zylindersegmentförmige Ausnehmungen (30', 33') zum Aufnehmen von zwei Stäben (22, 38) aufweisen.

6. Fixationseinrichtung nach einem der Ansprüche 3 bis 5, **dadurch gekennzeichnet, dass** das Unterteil (27') zwischen den zylindersegmentförmigen Ausnehmungen (30') und der kugelsegmentförmigen Ausnehmung (31) jeweils das Gewinde (29) zum Zusammenwirken mit den die beiden Teile verbindenden Schraubenelementen (36, 36') und das Oberteil (28') jeweils koaxial zu diesem Gewinde die Bohrung (35') aufweist.

7. Fixationseinrichtung nach einem der Ansprüche 3 bis 6, **dadurch gekennzeichnet, dass** das Oberteil (28') eine erste Hälfte (39) und eine zweite Hälfte (40) umfasst, die durch einen das Oberteil (28') in die beiden Teile (39, 40) teilenden Schlitz (41) getrennt sind.

8. Fixationseinrichtung nach einem der Ansprüche 1 bis 6, wobei das Oberteil (28') und das Unterteil (27') identisch ausgebildet und spiegelbildlich zueinander angeordnet sind.

9. Fixationseinrichtung mit wenigsten zwei Schrauben (21) nach einem der Ansprüche 3 bis 8 und zwei von den Schrauben zu erfassenden Stäben (22, 38).

10. Fixationseinrichtung mit wenigsten zwei Schrauben (21) nach einem der Ansprüche 1, 3 bis 8 und wenigsten einem von den Schrauben zu erfassenden Stab (22), die weiterhin eine Verbindungseinrichtung (50) aufweist zum Verbinden eines weiteren Stabs (51) mit dem Stab (22), wobei die Verbindungseinrichtung (50) aufweist:
einen als Schaft ausgebildeten Stab (52) mit jeweils einer Kugel (53, 54) an seinen beiden gegenüberliegenden Enden,
an jedem Ende des schaftförmigen Stabs (52) eine die Kugel (53, 54) aufnehmende Fassung (55, 56) aus zwei identisch ausgebildeten und spiegelbildlich zueinander angeordneten Teilen (27, 28), die zusammen einen Stab (22, 51) umfassen können, und
einem Schraubenelement (36) zum Verbinden der beiden Teile (27, 28) und gleichzeitigem Fixieren der Kugel (53, 54) und des Stabes (22, 51);
wobei die beiden Teile (27, 28)
in ihrer Mitte jeweils ein Gewinde (29, 35) zum Zusammenwirken mit dem die beiden Teile verbindenden Schraubenelement (36) aufweisen,
auf einer Seite einander zugewandte zylindersegmentförmige Ausnehmungen zum Aufnehmen des Stabes (22, 51) aufweisen,
auf der den zylindersegmentförmigen Ausnehmungen (30, 33) gegenüberliegenden Seite einander zugewandte kugelsegmentförmige Ausnehmungen (32, 34) zur Aufnahme der Kugel (53, 54) aufweisen, und
ausgehend von den kugelsegmentförmigen Ausnehmungen (32, 34) eine in der Mittelebene zwischen den Teilen (27, 28) zentrierte Ausnehmung (57) aufweisen zum Durchführen des schaftförmigen Stabs (52).

## Claims

1. Means for fixation of bones, in particular of a pelvis, comprising at least one screw (21) for a means for fixation, comprising:
a screw member (23) having a thread shaft (24) and a head (25) in the shape of a spherical segment,
a casing (26) accommodating the head (25) and having a lower part (27') facing the side of the shaft and an upper part (28; 28'; 28") which is adverted from the side of the shaft, which together may envelope a rod (22; 38), and
a screw member (36; 36') for connecting both parts (27', 28') and for fixing the screw member (23) with the head in the shape of a spherical segment and the rod (22; 38) at the same time; wherein
a thread (29, 35) for cooperating with the screw member (36; 36'), which connects both parts, is provided in the lower part (27'), and a bore (35; 35') is provided in the upper part (28') coaxial to the thread (29, 35);
at one side of the thread (29, 35) and the bore (35; 35'), recesses (30'; 33') in the shape of a cylinder segment, which are facing each other, are respectively provided for accommodating the rod (22; 38),
at the respectively opposite side of the thread (29, 35) and the bore (35; 35'), recesses (31, 34) in the shape of a spherical segment, which face each other, are provided for accommodating the spherical head (25),
**characterized in that**
between the recesses (31, 34) in the shape of a spherical segment and the outer surfaces of both parts (27', 28'), which are adverted from each other, recesses (32, 37) are provided for passing-through the thread shaft (24) on the one hand and a tool for rotating the screw member (23) with the head in the shape of a spherical segment on the other hand.

2. Fixation means having at least two screws (21) according to claim 1 and a rod (22; 38) to be grasped by the screws.

3. Fixation means according to claim 1, wherein
the casing (26) accommodating the head (25) with the lower part (27') facing the side of the shaft and the upper part (28') adverted from the side of the shaft may envelop a first rod (22) and a second rod (38), and wherein
two screw members (36, 36') are provided for connecting both parts (27', 28') and for fixing the screw member (23) with the head in the shape of a spherical segment and the rod (22, 38) at the same time.

4. Fixation means according to claim 3, **characterized in that** the lower part (27') and the upper part (28') respectively comprise in the centres thereof recesses (31, 34) which face each other for accommodating the spherical head (25), and are formed in a mirror-symmetrical manner to each other around a plane extending through the centre point of the recesses (34, 31) in the shape of a spherical segment and extending perpendicular to the plane between the upper part and the lower part and in parallel to the longitudinal axis of the rod (22).

5. Fixation means according to claim 3 or 4, **characterized in that** the lower part (27') and the upper part (28') comprise at both sides of the recesses (31, 34) in the shape of a spherical segment recesses (30', 33') in the shape of a cylinder segment for accommodating two rods (22, 38).

6. Fixation means according to one of claims 3 to 5, **characterized in that** the lower part (27') between the recesses (30') in the shape of a cylinder segment and the recess (31) in the shape of a spherical segment respectively comprise the thread (29) for cooperating with the screw members (36, 36'), which connect both parts, and that the upper part (28') respectively comprises the bore (35') coaxial to this thread.

7. Fixation means according to any one of claims 3 to 6, **characterized in that** the upper part (28') comprises a first half (39) and a second half (40), which are separated by a slit (41), which separates the upper part (28') in both parts (39, 40).

8. Fixation means according to any one of claims 1 to 6, wherein the upper part (28') and the lower part (27') are identically formed and arranged in a mirror-symmetrical manner to each other.

9. Fixation means comprising at least two screws (21) according to any one of claims 3 to 8 and two of the rods (22, 38) to be grasped by the screws.

10. Fixation means comprising at least two screws (21) according to any one of claims 1, 3 to 8 and comprising at least one of the rods (22) to be grasped by the screws, further comprising connection means (50) for connecting a further rod (51) to the rod (22), wherein the connection means (50) comprises:
a rod (52) formed as a shaft respectively having a sphere (53, 54) at both opposing ends thereof,
a casing (55, 56) accommodating the sphere (53, 54) at each end of the rod (52) in the shape of a shaft and consisting of two parts (27, 28), which are identically formed and arranged to each other in a mirror-symmetrical manner, which together may envelope a rod (22, 51), and
a screw member (36) for connecting both parts (27, 28) and fixing the sphere (53, 54) and the rod (22, 51) at the same time;
wherein both parts (27, 28)
respectively comprise in the centre thereof a thread (29, 35) for cooperating with the screw member (36) connecting both parts,
comprise at one side recesses in the shape of a cylinder segment, which face each other, for accommodating the rod (22, 51),
comprise recesses (32, 34) in the shape of a spherical segment for accommodating the sphere (53, 54) at the side opposite to the recesses (30, 33) in the shape of a cylinder segment, and
comprise, starting out from the recesses (32, 34) in the shape of a spherical segment, a recess (57) aligned to the centre plane between the parts (27, 28) for passing-through the rod (52) in the shape of a shaft.

## Revendications

1. Dispositif de fixation d'os et, en particulier, d'un bassin, avec au moins une vis (21) pour un dispositif de fixation comprenant :
un élément formant vis (23), avec une tige de vis (24) et une tête (25) en forme de segment de sphère,
une monture (26), recevant la tête (25), avec une partie inférieure (27'), tournée vers le côté tige, et une partie supérieure (28 ; 28' ; 28".), opposée au côté tige, pouvant enserrer conjointement une barre (22 ; 38), et
un élément formant vis (36 ; 36'), pour assurer la liaison des deux parties (27', 28') et la fixation simultanée de l'élément formant vis (23), à tête en forme de segment de sphère, et de la barre (22; 38) ; où
dans la partie inférieure (27'), un filetage (29, 35) est prévu pour coopérer avec l'élément formant vis (36 ; 36') reliant les deux parties et, dans la partie supérieure (28'), un perçage (35 ; 35') est prévu, coaxialement au filetage (29, 35) ;
chaque fois sur un côté du filetage (29, 35) et du perçage (35 ; 35') sont prévus des évidements (30' ; 33'), en forme de segments de cylindre, tournés les uns vers les autres, pour recevoir la barre (22 ; 38),
sur le côté, chaque fois opposé, du filetage (29, 35) et du perçage (35 ; 35') sont prévus des évidements (31, 34) en forme de segments de sphère, tournés les uns vers les autres, pour recevoir la tête sphérique (25),
**caractérisé en ce que**,
entre les évidements (31, 34), en forme de segments de sphère, et les faces de recouvrement, opposées les unes aux autres, des deux parties (27', 28') sont prévus des évidements (32, 37), pour le passage de la tige filetée (24), d'une part, et d'un outil pour faire tourner l'élément formant vis (23) à tête en forme de segment de sphère, d'autre part.

2. Dispositif de fixation avec au moins deux vis (21) selon la revendication 1 et une barre (22 ; 38) à saisir par les vis.

3. Dispositif de fixation selon la revendication 1, dans lequel
la monture (26), recevant la tête (25), avec la partie inférieure (27') tournée vers le côté tige et la partie supérieure (28') opposée au côté tige, peut enserrer une première barre (22) et une deuxième barre (38), et dans lequel
deux éléments formant vis (36, 36') sont prévus pour assurer la liaison des deux parties (27', 28') et la fixation simultanée de l'élément formant vis (23) à tête en forme de segment de sphère et des barres (22, 38).

4. Dispositif de fixation selon la revendication 3, **caractérisé en ce que** la partie inférieure (27') et la partie supérieure (28') présentent en chacun de leur centre des évidements (31, 34) en forme de segments de sphère, tournés les uns vers les autres, pour recevoir la tête sphérique (25), et sont réalisés en répondant en soi à une symétrie spéculaire, autour d'un plan passant par le centre des évidements (34, 31) en forme de segments de sphère, s'étendant perpendiculairement au plan entre partie supérieure et partie inférieure et parallèlement à l'axe longitudinal de la barre (22).

5. Dispositif de fixation selon la revendication 3 ou 4, **caractérisé en ce que** la partie inférieure (27') et la partie supérieure (28') présentent, de part et d'autre des évidements (31, 34) en forme de segments de sphère, des évidements (30', 33') en forme de segments de cylindre, pour recevoir deux barres (22, 38).

6. Dispositif de fixation selon l'une des revendications 3 à 5, **caractérisé en ce que** la partie inférieure (27') présente, entre les évidements (30') en forme de segments de cylindre et l'évidement (31) en forme de segments de sphère, chaque fois le filetage (29) pour coopérer avec les éléments formant vis (36 , 36') reliant les deux parties, et la partie supérieure (28') présente, chaque fois coaxialement à ce filetage, le perçage (35').

7. Dispositif de fixation selon l'une des revendications 3 à 6, **caractérisé en ce que** la partie supérieure (28') comprend une première moitié (39) et une deuxième moitié (40), séparées par une fente (41) divisant la partie supérieure (28') en les deux parties (39, 40).

8. Dispositif de fixation selon l'une des revendications 1 à 6, **caractérisé en ce que** la partie supérieure (28') et la partie inférieure (27') sont réalisées à l'identique et disposées l'une par rapport à l'autre en répondant à une symétrie spéculaire.

9. Dispositif de fixation avec au moins deux vis (21) selon l'une des revendications 3 à 8 et deux barres (22, 38) à saisir par les vis.

10. Dispositif de fixation avec au moins deux vis (21) selon l'une des revendications 1, 3 à 8 et au moins une barre (22) à saisir par les vis, présentant en plus un dispositif de liaison (50), pour assurer la liaison d'une autre barre (51) à la barre (22), le dispositif de liaison (50) présentant :
une barre (52), réalisée sous forme de tige, avec chaque fois une sphère (53, 54) à ses deux extrémités opposées,
à chaque extrémité de la barre (52) en forme de tige, une monture (55, 56), recevant les sphères (53, 54), composée de deux parties (27, 28), réalisées à l'identique et disposées en répondant à une symétrie spéculaire l'une par rapport à l'autre, les parties pouvant enserrer conjointement une barre (22, 51), et
un élément formant vis (36), pour assurer la liaison des deux parties (27, 28) et la fixation simultanée des sphères (53, 54) et de la barre (22, 51) ;
les deux parties (27, 28)
présentant chacune, en leur centre, un filetage (29, 35), pour coopérer avec l'élément formant vis (36) reliant les deux parties,
présentant, sur un côté, des évidements en forme de segments de cylindre, tournés les uns vers les autres, pour recevoir la barre (22, 51),
présentant, sur le côté opposé aux évidements (30, 33) en forme de segments de cylindre, des évidements (32, 34) en forme de segments de sphère, tournés les uns vers les autres, pour recevoir les sphères (53, 54), et
présentant, en partant des évidements (32, 34) en forme de segments de sphère, un évidement (57) centré dans le plan médian entre les parties (27, 28), pour le passage de la barre (52) en forme de tige.
